(19) Europäisches Patentamt
European Patent Office
Office européen des brevets

(11) **EP 1 390 541 B1**

(12) **EUROPÄISCHE PATENTSCHRIFT**

(45) Veröffentlichungstag und Bekanntmachung des Hinweises auf die Patenterteilung:
**26.10.2005 Patentblatt 2005/43**

(21) Anmeldenummer: 02737935.3

(22) Anmeldetag: **09.04.2002**

(51) Int Cl.$^7$: **C12Q 1/68**

(86) Internationale Anmeldenummer:
**PCT/EP2002/003956**

(87) Internationale Veröffentlichungsnummer:
**WO 2002/097126 (05.12.2002 Gazette 2002/49)**

(54) **VERFAHREN ZUM NACHWEIS VON GRAM-POSITIVEN BAKTERIEN**

METHOD FOR DETECTING GRAM-POSITIVE BACTERIA

PROCEDE DE DETECTION DE BACTERIES GRAM POSITIF

(84) Benannte Vertragsstaaten:
**AT BE CH CY DE DK ES FI FR GB GR IE IT LI LU MC NL PT SE TR**

(30) Priorität: **03.05.2001 DE 10121505**

(43) Veröffentlichungstag der Anmeldung:
**25.02.2004 Patentblatt 2004/09**

(73) Patentinhaber: **Hain Lifescience GmbH**
**72147 Nehren (DE)**

(72) Erfinder: **WEIZENEGGER, Michael**
**69168 Wiesloch (DE)**

(74) Vertreter: **Keller, Günter et al**
**Lederer & Keller**
**Patentanwälte**
**Prinzregentenstrasse 16**
**80538 München (DE)**

(56) Entgegenhaltungen:
US-A- 5 703 217

• ROLLER C ET AL: "GRAM-POSITIVE BACTERIA WITH A HIGH DNA G+C CONTENT ARE CHARACTERIZED BY A COMMON INSERTION WITHIN THEIR 23S RRNA GENES" JOURNAL OF GENERAL MICROBIOLOGY, SOCIETY FOR MICROBIOLOGY, READING, GB, Bd. 138, Nr. 6, 1992, Seiten 1167-1175, XP001120764 ISSN: 0022-1287

• ROLLER C ET AL: "IN SITU PROBING OF GRAM-POSITIVE BACTERIA WITH HIGH DNA G+C CONTENTUSING 235RRNA-TARGETED OLIGONUCLEOTIDES" MICROBIOLOGY, SOCIETY FOR GENERAL MICROBIOLOGY, READING, GB, Bd. 140, Nr. 10, 1. Oktober 1994 (1994-10-01), Seiten 2849-2858, XP000563032 ISSN: 1350-0872

• DATABASE EMBL [Online] EBI; 24. Mai 1996 (1996-05-24) COLE S.T. ET AL.: "Mycobacterium tuberculosis H37Rv complete genome; segment 59/162" retrieved from HTTP://WWW.EBI.AC.UK Database accession no. Z73902 XP002228144

• HUEGLE B ET AL: "A CLOSER LOOK AT THE INSERTION WITHIN HELIX 54 OF THE 23S-RRNA OF THE GENUS CORYNBACTERIUM (AND RELATED TAXA)" CLINICAL LABORATORY, CLIN LAB PUBLICATIONS, HEIDELBERG, DE, Bd. 46, Nr. 5/6, 2000, Seiten 255-260, XP009002062 ISSN: 1433-6510

• FU L M ET AL: "IS MYCOBACTERIUM TUBERCULOSIS A CLOSER RELATIVE TO GRAM-POSITIVE OR GRAM-NEGATIVE BACTERIAL PATHOGENS?" TUBERCULOSIS, ELSEVIER, GB, Bd. 82, Nr. 2/3, 2002, Seiten 85-90, XP009002056 ISSN: 1472-9792

**Beschreibung**

Die vorliegende Erfindung betrifft das Gebiet der Diagnostik von Mikroorganismen, insbesondere den Nachweis von Gram-positiven Bakterien mit hohem GC-Gehalt wie Mykobakterien in der klinischen Diagnostik.

Der Nachweis von Bakterien und ihre genaue Identifizierung spielten eine sehr wichtige Rolle in der klinischen Diagnostik. So sollte ein bestimmter Erreger bei bakteriellen Infektionen möglichst schnell identifiziert werden, damit eine entsprechende Behandlung eingeleitet werden kann.

Eine wichtige Rolle spielen dabei Gram-positive Bakterien, insbesondere Mykobakterien. Gegenwärtig sind über 100 Mykobakterienspezies bekannt, die aus klinischem Material nachgewiesen und differenziert wurden. Von besonderer Bedeutung sind hierbei pathogene Mykobakterienspezies wie der *Mycobacterium tuberculosis*-Komplex, *Mycobacterium intracellulare* und *Mycobacterium avium*.

In der medizinischen Routinediagnostik sowie in der Lebensmittelanalytik erfolgte die klassische Identifizierung von Bakterien über Selektivmedien und anschließende Untersuchung der biochemischen Eigenschaften. Dabei läßt sich aber oftmals nicht die genaue Spezies bestimmen. Zudem sind diese Untersuchungen sehr zweitaufwendig, und es muß für die weitergehenden Untersuchungen eine Reinkultur vorliegen. Gerade Bakteriengruppen von hoher Komplexität, großer Diversität und schwierigen Wachstumsbedingungen sind der klassischen Kulturdifferenzierung schwer zugänglich und/oder verzögern durch ihre langsames Wachstum die Diagnostik erheblich.

In den letzten Jahren wurden zunehmend Nukleinsäure-basierende Verfahren eingeführt, um Bakterien nachzuweisen. Dabei werden beispielsweise mit Spezies-spezifischen Primern Nukleinsäureamplifikationsreaktionen durchgeführt. Die Detektion erfolgt meist über Gelelektrophorese oder über immobilisierte Sonden in Mikrotiterplatten. Solche Techniken eignen sich aber nicht, um aus vielen möglichen pathogenen Organismen einen oder mehrere nachzuweisen. Ein Ansatz für diese Fragestellung ist ein Amplifikationsansatz mit einem Gemisch verschiedener Speziesspezifischer Amplifikationsprimer und entsprechenden Sonden und/oder ein Primerpaar, das komplementär zu einem Basenabschnitt einer Gruppe von Organismen ist. Die Speziesspezifität wird hier über die Sondenstruktur gewährleistet.

Als Zielsequenz zur Amplifizierung Bakterien-spezifischer Nukleinsäure wurde bereits die ribosomale RNA (rRNA) oder die ribosomale DNA (rDNA) inklusive ihrer Spacerstrukturen verwendet. Die diagnostisch am häufigsten verwendete Zielsequenz ist die 16S rRNA. Sie ist in den entsprechenden Datenbanken am besten repräsentiert. Durch den relativ konservierten Charakter finden sich aber nicht immer Spezies-charakteristische Sequenzstrukturen. Im Unterschied dazu ist die 16S-23S rDNA-Spacerregion innerhalb vieler Spezies hochvariabel und eignet sich gut zur Identifizierung von Bakterien. Sie ist in ihrem Strukturinformationsgehalt der 16 S rRNA auch überlegen. Auch diese Zielregionen eignen sich aber nur bedingt, um zwischen einer großen Anzahl von Mykobakterienspezies zu unterscheiden, da ihre Sequenzvariabilität zu hoch ist und damit die Sensitivität für Sonden gegen diese Zielregion abnimmt.

Liesack et al. (1991) FEBS Lett. 281, 114-118 offenbaren die komplette Nukleotidsequenz der 23S und 5S rRNA von *Mycobacterium leprae* und verschiedene Zielsequenzen zum Nachweis von Bakterien. Unter anderem wurde eine Sonde aus dem Bereich der Helix 54 zum Nachweis von *Mycobacterium leprae* offenbart. Daraus läßt sich jedoch nicht ableiten, daß es anhand der Helix 54-Zielregion möglich ist, zwischen einer sehr großen Anzahl verschiedener Spezies von Gram-positiven Bakterien mit hohem GC-Gehalt, z.B. Mykobakterien, zu unterscheiden.

Roller et al., J. of General Microbiology, Oktober 1992, S. 1167-1175 beschreiben den phylogenetischen Zusammenhang der Helix 54-Insertion innerhalb der gram-positiven Bakterien mit hohem G + C-Gehalt und zeigen, dass diese Insertion auch in der reifen 23S rRNS von Corynebacterium glutamicum vorhanden ist. In dieser Literaturstelle wird kein Nachweisverfahren bzw. Diagnostizierverfahren offenbart und es werden auch keinerlei Vorschläge für Primer- oder Sondensequenzen gemacht. Die Anwendung der variablen Insertion als diagnostische Zielsequenz wird in dieser Literaturstelle nicht offenbart.

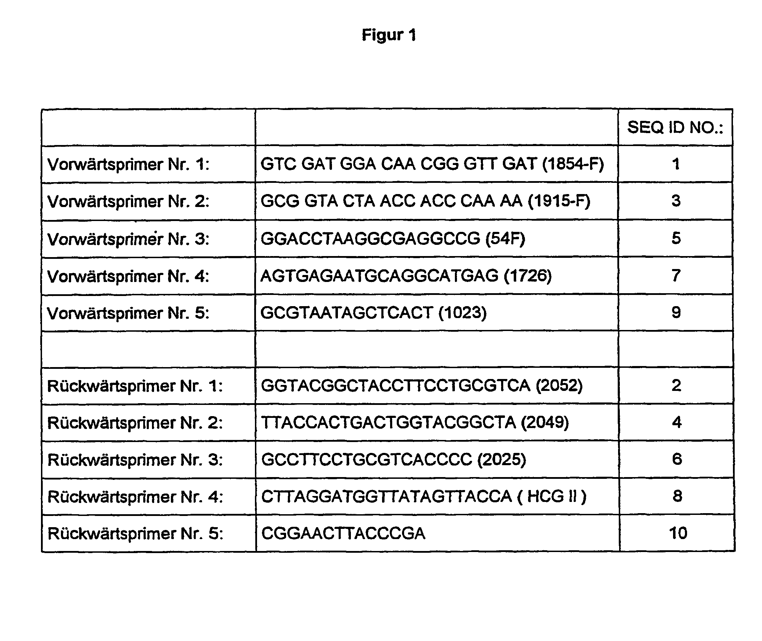

Roller et al., Microbiology (1994), S. 2849-2858 beschreiben Sonden zur "in situ"-Hybridisierung bei grampositiven Bakterien mit hohem G + C-Gehalt, um die Einheitlichkeit dieser Gruppe durch homologe 23S rRNS-Strukturen aufzuzeigen. Die dort untersuchte Sequenz liegt nicht in der Helix 54, sondern in der Helix 69 der 23S rRNS Domäne IV (siehe Figur 1, S. 2852).

Das US-Patent Nr. 5 703 217 beschreibt Polymorphismen zur Differenzierung von Mykobakterienspezies mit der 23S rRNS als Zielsequenz. Zwar wurden für einige wenige Spezies experimentelle Beispiele vorgelegt. Die vorgeschlagenen Sondensequenzen sind aber nicht geeignet, um hieraus eine praktische Anwendung zur Differenzierung innerhalb des Genus Mycobakterium abzuleiten. Da die offenbarten Sequenzen zum Teil sehr lang sind, muss mit einem erheblichen Verlust an Spezifität und hoher Kreuzreaktivität gerechnet werden.

Keines der Dokumente des relevanten Standes der Technik offenbart ein Verfahren zum Nachweis und zur Identifikation von Gram-positiven Bakterien mit hohem GC-Gehalt, bei dem die Region der Helix 54 unter Verwendung von geeigneten Primern mit den Sequenzen amplifiziert wird.

Die vorliegende Erfindung betrifft ein Verfahren zum Nachweis und/oder zur Identifizierung von Gram-positiven Bakterien mit hohem GC-Gehalt, gemäß dem zunächst eine Nukleinsäureamplifikationsreaktion durchgeführt wird und anschließend eine Zusammensetzung enthaltend das Amplifikationsprodukt oder einen Teil davon mit einer oder mehreren Sonden hybridisiert wird. Schließlich wird das Ausmaß der Hybridisierung bestimmt.

Als Nukleinsäureamplifikationsreaktion können verschiedene Reaktionen eingesetzt werden. Bevorzugt wird die Polymerasekettenreaktion (PCR) eingesetzt. Die verschiedenen Ausgestaltungen der PCR-Technik sind dem Fachmann bekannt, siehe z.B. Mullis (1990) Target amplification for DNA analysis by the polymerase chain reaction. Ann Biol Chem (Paris) 48(8), 579-582. Weitere Amplifikationstechniken, die zur Anwendung kommen können, sind "nucleic acid strand-based amplification" (NASBA), "transcriptase mediated amplification" (TMA), "reverse transcriptase polymerase chain reaction" (RT-PCR), "Q-β replicase amplification" (β-Q-Replicase) und die "single strand displacement amplification" (SDA). NASBA und andere Transkriptions-basierte Amplifikationsmethoden werden in Chan und Fox, Reviews in Medical Microbiology (1999), 10 (4), 185-196 erläutert.

Die Nukleinsäureamplifikationsreaktion wird mit einer Probenzusammensetzung durchgeführt. Die Probenzusammensetzung kann jede Zusammensetzung sein, die im Verdacht steht, Bakterien zu enthalten, insbesondere Gram-positive Bakterien mit hohem GC-Gehalt. Es kann sich um Primärmaterial, z.B. Trachealsekrete, Wundabstriche, Blut usw. handeln oder um Kulturen von Mikroorganismen, die bereits in Flüssig- oder auf Festmedien angezogen wurden.

Als Primerpaar in der Nukleinsäureamplifikationsreaktion ist erfindungsgemäß ein erster Primer mit einer der Sequenzen SEQ ID NO. 1, SEQ ID NO. 3, SEQ ID NO. 5, SEQ ID NO. 7 oder SEQ ID NO. 9 und ein zweiter Primer mit einer der Sequenzen SEQ ID NO. 2, SEQ ID NO. 4, SEQ ID NO. 6, SEQ ID NO. 8 oder SEQ ID NO. 10 anwesend. Die Sequenzen der einzelnen Primer sind in Figur 1 dargestellt.

In einer bevorzugten Ausführungsform ist in der Nukleinsäureamplifikationsreaktion eines der Primerpaare mit den Sequenzen

SEQ ID NO. 1/SEQ ID NO. 2 (Primerpaar 1),
SEQ ID NO. 3/SEQ ID NO. 4 (Primerpaar 2),
SEQ ID NO. 5/SEQ ID NO. 6 (Primerpaar 3),
SEQ ID NO. 7/SEQ ID NO. 8 (Primerpaar 4) und
SEQ ID NO. 9/SEQ ID NO. 10 (Primerpaar 5)
anwesend.

Jedes der Primerpaare kann zur Amplifikation eines Nukleinsäurebereichs, der wenigstens einen Teil der Helix 54 der 23S rDNA/rRNA von Gram-positiven Bakterien mit hohem GC-Gehalt umfaßt, verwendet werden. Die Primer können in unterschiedlicher Kombination den Helix 54-Bereich aller G+C-reichen Gram-positiven Bakterien amplifizieren. Besonders Primer SEQ ID NO. 8 (Figur 1) ist in der Lage bei geeigneten Reaktionsbedingungen spezifisch nur an Nukleinsäure von G+C-reichen Gram-positiven Bakterien zu binden. Die Kombination der Primer SEQ ID NO. 3 (Figur 1) mit SEQ ID NO. 2 (Figur 1) oder mit SEQ ID NO. 4 (Figur 1) amptifiziert hochspezifisch *M. chelonae-Komplex, M. kansasii, M. malmoense, M. tuberculosis-Komplex, M. avium, M. scrophulaceum und M. interjectum.*

Dem Fachmann ist bewußt, daß ausgehend von der Lehre der vorliegenden Erfindung auch Primer entworfen werden können, die geringfügig von den erfindungsgemäßen Primem abweichen, aber dennoch funktionieren. So sind auch Primer denkbar, die gegenüber den erfindungsgemäßen Primem mit den Sequenzen SEQ ID NO. 1 bis 10 am 5'- und/oder 3'-Ende Verlängerungen bzw. Verkürzungen um wenigstens ein, zwei oder drei Nukleotide aufweisen. Insbesondere Verlängerungen oder Verkürzungen am 5'-Ende der Primer können immer noch funktionsfähige Primer liefern, die erfindungsgemäß eingesetzt werden können. Ebenso ist es denkbar, daß einzelne oder wenige Nukleotide eines Primers durch andere Nukleotide ausgetauscht sind, solange die Spezifität der Primer nicht zu stark verändert wird und die Schmelztemperatur der Primer nicht zu stark verändert wird. Dem Fachmann ist klar, daß neben den üblichen Nukleotiden A, G, C und T auch modifizierte Nukleotide wie Inosin usw. zur Anwendung kommen können. Die Lehre der vorliegenden Erfindung ermöglicht solche Modifikationen, ausgehend vom Gegenstand der Ansprüche.

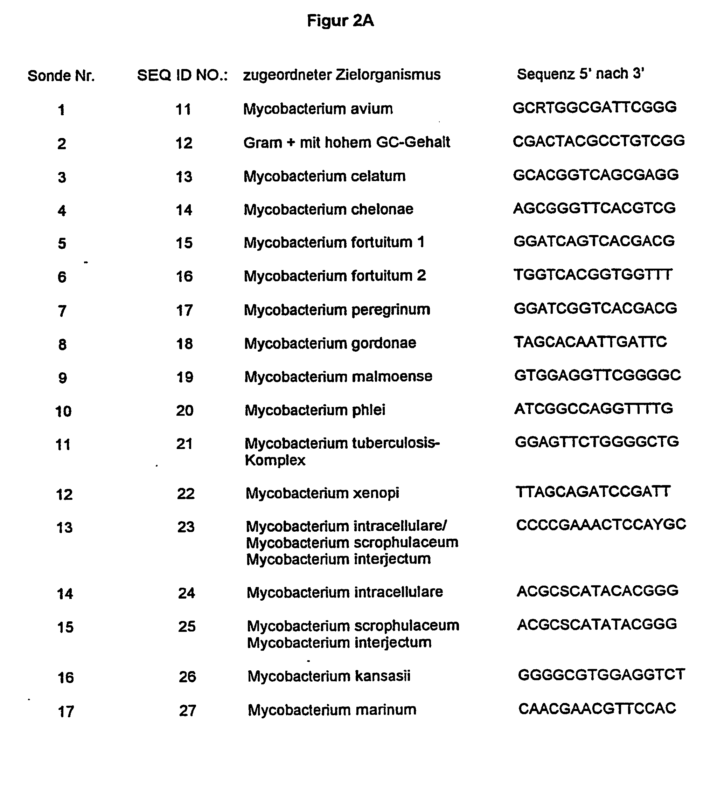

Erfindungsgemäß wird eine Zusammensetzung, die das Amplifikationsprodukt oder einen Teil davon enthält, mit einer oder mehreren Sonden hybridisiert. Die Sonden sind Oligonukleotide, die eine der Sequenzen SEQ ID NO. 11 bis 42 oder der dazu komplementären Sequenzen haben. Die Sequenzen der einzelnen Sonden sind in Figur 2A und 28 dargestellt. Auch bei den Sonden sind geringfügige Abwandlungen denkbar, ohne daß ihre Funktionalität wesentlich beeinträchtigt wird. Die Sonden der vorliegenden Erfindung können am 5'-Ende und/oder am 3'-Ende um wenige Nukleotide verlängert und/oder verkürzt sein, bevorzugt um höchstens drei Nukleotide, bevorzugter um höchstens zwei Nukleotide, am bevorzugtesten um ein Nukleotid. Es ist ebenfalls denkbar, daß ein oder wenige Nukleotide in der Sequenz der erfindungsgemäßen Sonden gegen ein anderes Nukleotid ausgetauscht wird, solange die Hybridisierung an die Zielsequenz noch möglich ist. Das schließt ein, daß bei Abwandlungen die Schmelztemperatur der abgewandelten Sonde nicht zu stark von der Schmelztemperatur der ursprünglichen Sonde abweicht. Die Schmelztemperatur wird dabei nach der Formel:

$$\text{Schmelztemperatur} = (\text{Anzahl der G/C-Basenpaare}) \cdot 4°C +$$

+ (Anzahl der A/T-Basenpaare) • 2°C

berechnet.

Prinzipiell ist es möglich, daß durch Hybridisierung mit einer einzigen spezifischen Sonde die Bakterienspezies bestimmt wird. Es ist aber auch möglich, die Zusammensetzung, die das Amplifikationsprodukt oder einen Teil davon enthält, mit mehr als einer Sonde zu hybridisieren. Dadurch wird die Aussagekraft des Verfahrens erhöht. Die genaueste Aussage ist möglich, wenn eine Hybridisierung mit allen Sonden mit den Sequenzen SEQ ID NO. 11 bis 42 (oder den dazu komplementären Sequenzen) durchgeführt wird. Man erhält dann ein genaues Profil und kann die Bakterienspezies mit hoher Sicherheit bestimmen. So hybridisiert z.B. Sonde SEQ ID NO. 23 (Figur 2A) mit Nukleinsäure aus den Bakterien *M. intracellulare, M. scrophulaceum* und *M. interjectum. M. Intracellulare* kann aber durch Sonde SEQ ID NO. 24 (Figur 2A) von *M. scrophulaceum* und *M. interjectum* abgetrennt werden. Sonde SEQ ID NO. 25 (Figur 2A) hybridisiert mit Nukleinsäure aus *M. celatum, M. chelonae-Komplex, M. gordonae, M. kansasii, M. malmoense, M. tuberculosis-Komplex, M. scrophulaceum* und *M. interjectum* und anderen Mykobakterinspezies, aber soweit bekannt nur mit Arten der Gattung Mykobakterien. Zusammen mit SEQ ID NO. 23 (Figur 2A) trennt sie die Arten *M. scrophulaceum* und *M. interjectum* nach heutigem Stand des Wissens von allen anderen Mykobakterien ab. Ähnliches gilt für Sonde SEQ ID NO. 24.

Die Hybridisierung findet üblicherweise so statt, daß entweder die Zusammensetzung, die das Amplifikationsprodukt oder einen Teil davon enthält, oder die Sonde auf einer festen Phase immobilisiert wird und mit dem jeweils anderen Hybridisierungspartner in Kontakt gebracht wird. Als feste Phasen sind verschiedenste Materialien vorstellbar, beispielsweise Nylon, Nitrocellulose, Polystyrol, silikatische Materialien usw. Es ist auch denkbar, daß als feste Phase eine Mikrotiterplatte eingesetzt wird.

In der Regel ist wenigstens eine Sonde oder wenigstens ein Primer markiert. Verschiedenste Markierungen sind dabei denkbar, wie z.B. Fluoreszenzfarbstoffe, Biotin oder Digoxigenin. Bekannte Fluoreszenzmarkierungen sind Fluoreszein, FITC, Cyaninfarbstoffe usw. Die Markierungen sind üblicherweise kovalent mit den Oligonukleotiden verbunden. Während eine Fluoreszenzmarkierung direkt nachgewiesen werden kann, können Biotin- und Digoxigeninmarkierungen nach Inkubation mit geeigneten Bindemolekülen nachgewiesen werden. Beispielsweise kann ein Biotinmarkiertes Oligonukleotid nachgewiesen werden, indem es mit einer Lösung in Kontakt gebracht wird, die Streptavidin gekoppelt an ein Enzym enthält, wobei das Enzym, z.B. Peroxidase oder alkalische Phosphatase, ein Substrat umsetzt, das einen Farbstoff erzeugt oder zu Chemilumineszenz führt. Derartige Verfahren sind dem Fachmann an sich bekannt.

In einer Ausführungsform der Erfindung ist wenigstens einer der verwendeten Primer markiert. Vorzugsweise sind mehrere der in der Reaktion anwesenden Primer markiert. Werden markierte Primer verwendet, dann sind die Sonden in der Regel nicht markiert. In dieser Ausführungsform des Verfahrens werden die Sonden auf einer festen Phase immobilisiert, und anschließend wird diese feste Phase mit der Zusammensetzung, die das Amplifikationsprodukt oder einen Teil davon enthält, in Kontakt gebracht: Der Vorteil dieses Verfahrens liegt darin, daß auf der festen Phase mehr als eine Sonde immobilisiert werden kann. Vorzugsweise werden wenigstens zwei Sonden auf der festen Phase immobilisiert, bevorzugter wenigstens fünf Sonden, noch bevorzugter wenigstens zehn Sonden, am bevorzugtesten die Sonden mit den Sequenzen SEQ ID NO. 11 bis 42 oder den entsprechend komplementären Sequenzen. Durch Inkubation des Amplifikationsprodukts oder eines Teils davon mit einer derart vorbereiteten festen Phase mit immobilisierten Sonden kann durch einen einzigen Hybridisierungsschritt eine Aussage über die Hybridisierung des Amplifikationsprodukts mit allen immobilisierten Sonden gewonnen werden. Die feste Phase ist daher bevorzugt ein Mikroarray von immobilisierten Sonden auf einer festen Phase. Derartige "DNA-Chips" erlauben es, daß auf einem kleinen Bereich eine hohe Anzahl verschiedener Oligonukleotide immobilisiert werden. Die festen Phasen, die für DNA-Chips geeignet sind, bestehen vorzugsweise aus silikatischen Materialien wie Glas usw. Die Markierung der Primer ist in dieser Ausführungsform vorzugsweise eine Fluoreszenzmarkierung. Der DNA-Chip kann nach Inkubation mit dem Amplifikationsprodukt durch eine Scanvorrichtung rasch analysiert werden. Derartige Vorrichtungen sind dem Fachmann bekannt. Eine Übersicht über die Chip-Technologie gibt McGlennen (2001) Miniaturization technologies for molecular diagnostics. Clin Chem 47(3), 393-402.

In einer anderen Ausführungsform weist wenigstens eine der Sonden eine Markierung auf. Üblicherweise wird dann die Zusammensetzung, die das Amplifikationsprodukt oder einen Teil davon enthält, auf einer festen Phasen immobilisiert und mit einer Zusammensetzung in Kontakt gebracht, die wenigstens eine Sonde enthält, die ausgewählt ist aus der Gruppe bestehend aus Sonden mit den Sequenzen SEQ ID NO. 11 bis 42 und dazu komplementären Sequenzen. Auch in dieser Ausführungsform ist es bevorzugt, eine Hybridisierung mit mehr als einer Sonde durchzuführen. Dazu können mehrere feste Phasen bereitgestellt werden, auf denen das Amplifikationsprodukt immobilisiert ist. Es ist aber auch möglich, auf einer festen Phase an mehreren räumlich voneinander getrennten Bereichen kleine Mengen des Amplifikationsprodukts zu immobilisieren. Diese verschiedenen Spots werden dann mit jeweils verschiedenen Sonden in Kontakt gebracht (Hybridisierung).

Die Durchführung des Hybridisierungsverfahrens ist dem Fachmann an sich bekannt. So werden üblicherweise die festen Phasen nach Inkubation mit der Lösung, die den Hybridisierungspartner enthalten kann, stringenten Bedingungen ausgesetzt, um unspezifisch gebundene Nukleinsäuremoleküle zu entfernen. Die Hybridisierung kann in herkömmlicher Weise auf einer Nylon- oder Nitrocellulosemembran durchgeführt werden wie beschrieben (Sambrook et al., Molecular Cloning, Cold Spring Harbor Laboratory, 1989). Die darin genannten Prinzipien lassen sich vom Fachmann auf weitere Ausführungsformen übertragen.

Erfindungsgemäß wird nach der Hybridisierung das Ausmaß der Hybridisierung bestimmt. Das erfolgt üblicherweise dadurch, daß die Menge der Markierung bestimmt wird, die an die feste Phase gebunden ist. Derartige Nachweisreaktionen und Detektionsverfahren sind dem Fachmann an sich bekannt.

Offenbart werden Oligonukleotide, die eine der Sequenzen SEQ ID NO. 1 bis 42 haben sowie Oligonukleotide, die Sequenzen haben, die komplementär zu einer der Sequenzen SEQ ID NO. 11 bis 42 sind.

Weiterhin werden Zusammensetzungen offenbart enthaltend wenigstens ein derartiges Oligonukleotid. Vorzugsweise enthält die Zusammensetzung ein Primerpaar, wobei der erste Primer ausgewählt ist aus der Gruppe bestehend aus den Primem mit den Sequenzen SEQ ID NO. 1, SEQ ID NO. 3, SEQ ID NO. 5, SEQ ID NO. 7 und SEQ ID NO. 9, und der zweite Primer ausgewählt ist aus der Gruppe bestehend aus den Primem mit den Sequenzen SEQ ID NO. 2, SEQ ID NO. 4, SEQ ID NO. 6, SEQ ID NO. 8 und SEQ ID NO. 10. Bevorzugter enthält die Zusammensetzung eines der Primerpaare mit den Sequenzen

SEQ ID NO. 1/SEQ ID NO. 2 (Primerpaar 1),
SEQ ID NO. 3/SEQ ID NO. 4 (Primerpaar 2),
SEQ ID NO. 5/SEQ ID NO. 6 (Primerpaar 3),
SEQ ID NO. 7/SEQ ID NO. 8 (Primerpaar 4) und
SEQ ID NO. 9/SEQ ID NO. 10 (Primerpaar 5).

Offenbart wird eine feste Phase, auf der wenigstens ein Oligonukleotid mit einer der Sequenzen SEQ ID NO. 11 bis 42 oder einer dazu komplementären Sequenz immobilisiert ist. Wenn mehrere Oligonukleotide immobilisiert sind, sind diese auf der festen Phase räumlich voneinander getrennt. Ein weiterer Aspekt der Erfindung sind auf der festen Phase die Oligonukleotide mit den Sequenzen SEQ ID NO. 11 bis 42 oder einer dazu komplementären Sequenz immobilisiert, wobei die feste Phase als DNA-Chip ausgebildet ist.

Weiterhin betrifft die Erfindung ein Kit zum Nachweis und/oder zur Identifizierung von Gram-positiven Bakterien mit hohem GC-Gehalt, insbesondere Mykobakterien, enthaltend wenigstens ein Primerpaar, wobei der erste Primer ausgewählt ist aus der Gruppe bestehend aus den Primern mit den Sequenzen SEQ ID NO. 1, SEQ ID NO. 3, SEQ ID NO. 5, SEQ ID NO. 7 und SEQ ID NO. 9, und der zweite Primer ausgewählt ist aus der Gruppe bestehend aus den Primem mit den Sequenzen SEQ ID NO. 2, SEQ ID NO. 4, SEQ ID NO. 6, SEQ ID NO. 8 und SEQ ID NO. 10; und wenigstens ein Oligonukleotid mit einer der Sequenzen SEQ ID NO. 11 bis 42 oder der dazu komplementären Sequenzen. Bevorzugter enthält das Kit eines der Primerpaare mit den Sequenzen

SEQ ID NO. 1/SEQ ID NO. 2 (Primerpaar 1),
SEQ ID NO. 3/SEQ ID NO. 4 (Primerpaar 2),
SEQ ID NO. 5/SEQ ID NO. 6 (Primerpaar 3),
SEQ ID NO. 7/SEQ ID NO. 8 (Primerpaar 4) und
SEQ ID NO. 9/SEQ ID NO. 10 (Primerpaar 5).

Die bevorzugten Ausführungsformen des DNA-Chips und der Kits der vorliegenden Erfindung entsprechen denen des erfindungsgemäßen Verfahrens.

Schließlich betrifft die Erfindung auch die Verwendung eines oder mehrerer der Oligonukleotide mit den Sequenzen SEQ ID NO. 1 bis 10 als Primer in einer Nukleinsäureamplifikationsreaktion zum Nachweis und/oder zur Identifizierung von Gram-positiven Bakterien mit hohem GC-Gehalt.

Die Erfindung betrifft auch die Verwendung eines oder mehrerer der Oligonukleotide mit den Sequenzen SEQ ID NO. 11 bis 42 oder dazu komplementären Sequenzen als Sonde in einer Hybridisierungsreaktion, zum Nachweis und/oder zur Identifizierung von Gram-positiven Bakterien mit hohem GC-Gehalt.

Die hier beschriebenen 23S rRNA/rDNA-Sonden und ihre Anwendung zur Erkennung und Differenzierung von Bakterien des phylogentischen Astes der Gram-positiven Bakterien mit hohem GC-Gehalt haben große Bedeutung in der medizinischen Mikrobiologie. Diese Spezies sind in der Regel mit morphologisch oder biochemischen Methoden sehr schwer differenzierbar und/oder aufgrund ihres langsamen Wachstums verzögern sich die Identifizierung erheblich. Durch konservative flankierende Bereiche ist diese Genregion mit einem einzigen Set von Primem (generische Amplifikationsprimer) einer Nukleinsäureamplifikation zugänglich. Dadurch ist der Einsatz der Chiptechnologie möglich.

Figur 1 zeigt die Nukleinsäuresequenzen der Primer mit den Sequenzen SEQ ID NO. 1 bis 10.

Figur 2A zeigt eine Zusammenstellung der Sonden mit den Sequenzen SEQ ID NO. 11 bis 27 und die jeweils zugeordneten Zielorganismen.

Figur 2B zeigt die Sequenzen der Sonden mit den Sequenzen SEQ ID NO. 28 bis 42 und die jeweils zugeordneten Zielorganismen.

Die nachfolgenden Beispiele sollen die Erfindung näher erläutern.

**Beispiel 1**

*DNA/RNA-Isolierung:*

Bakterielle Nukleinsäure wurde entweder von Festnährmedien, Flüssigmedien oder aus Primärmaterial nach entsprechender Vorbehandlung gewonnen.

Tabelle 1:

| Bakterienspezies, die in Beispiel 1 untersucht wurden | | |
|---|---|---|
| **Spezies** | **Abkürzung** | **ATCC Nr.** |
| Mycobacterium avium | M.avi | 25291 |
| Mycobacterium celatum | M.cel | |
| Mycobacterium chelonae | M.che | 14472 |
| Mycobacterium fortuitum | M.fo1 | 6841 |
| Mycobacterium fortuitum | M.fo2 | |
| Mycobacterium peregrinum | M.per | |
| Mycobacterium gordonae | M.gor | 14470 |
| Mycobacterium malmoense | M.mal | 29571 |
| Mycobacterium phlei | M.phl | 11758 |
| Mycobacterium tuberculosis | M.tub | 27294 25177 |
| Mycobacterium xenopi | M.xen | 19971 |
| Mycobacterium intracellulare | M.int | 13950 |
| Mycobacterium scrophulaceum | M.scr | 19981 |
| Mycobacterium interjectum | M.itm | |
| Mycobacterium kansasii | M.kan | 12478 |
| Mycobacterium marinum | M.mar | 827 |
| Escherichia coli | E.col | |
| Humane DNA | hDNA | |
| Negativkontrolle | N-Kon | |

Dazu wurde von Festmedien mit einer sterilen Impföse bakterielles Material entnommen und in 300µl 10mM Tris/HCl pH 7,5 suspendiert. Aus Flüssigkulturen wurde 1ml entnommen, 5min bei 13.000rpm in einer Tischzentrifuge zentrifugiert, der Überstand verworfen und in 300µl 10mM Tris/HCl pH 7,5 resuspendiert. Primärmaterial wurde mit Acetylcystein verflüssigt und mit NaOH/SDS "dekontaminiert". Die so erhaltenen Zellsuspensionen wurden 15min bei 95°C in einem Thermomixer (Eppendorf, Hamburg, Deutschland) inkubiert, 15min in einem Ultraschallbad (Bandelin) beschallt und 10min bei 13.000rpm in einer Tischzentrifuge zentrifugiert. Vom Überstand wurden jeweils 5µl in die Amplifikationsreaktion eingesetzt.

*Amplifikation:*

Alle Primer wurden kommerziell synthetisiert (Interactiva, Ulm, Deutschland). Der PCR-Ansatz enthielt 1 x Taq-Puffer (Qiagen, Hilden, Deutschland), je 1µM Primer, 200µM dNTP (Roche) und 1U Hotstar Taq-Polymerase (Qiagen, Hilden, Deutschland). Die PCR-Amplifikation wurde auf einem Thermocycler PE 9600 (ABI, Weiterstadt, Deutschland) mit 15min 95°C, 10 Zyklen mit 30sek 95°C und 2min 60°C und mit 20 Zyklen 10sek 95°C, 50sek 55°C und 30sek 70°C durchgeführt.

Für die RNA-Amplifikation mit der NASBA-Technik wurde das NuctiSens Amplifikationskit (Organon Technika, Boxtel, Niederlande) nach Angaben des Herstellers verwendet:

1. Herstellung des Amplifikationsmixes: 8μl "reagent sphere" in "reagent dilution"-Puffer gelöst (enthält die für die Reaktion benötigten Enzyme), 5μl KCl-Lösung, Endkonzentration 70mM KCl und 2μl Primerlösung, Endkonzentration 0,5μM Primer;
2. 5μl RNA-Lösung zugeben und 60min bei 41 °C im Wasserbad inkubieren.

DNA/RNA-Amplifikat wurde entweder mit einem ethidiumbromidgefärbtem Agarosegel oder durch Hybridisierung nachgewiesen.

*Detektion der Amplifikate durch Sondenhybridisierung:*

Alle Sonden wurden am 5'-Ende biotiniliert, um Zielsequenz/Sonden-Hybride über an Streptavidin gekoppelte Reporterenzyme nachweisen zu können. Als Sonden werden Oligonukleotide mit den Sequenzen SEQ ID NO. 11 bis 27 eingesetzt (siehe Figur 2A).

Saugfähiges Papier (Blotting Papier GB002, Schleicher & Schüll, Dassel, Deutschland), und eine Nylonmembran (Biodyne A, Pall, Portsmouth, England) wurden auf die Größe der Blotapparatur (Minifold Schleicher & Schüll, Dassel, Deutschland) geschnitten und mit 10 x SSC getränkt. In die Öffnungen der zusammengebauten Apparatur wurden 250μl Denaturierungslösung (50 mM NaOH; 1,5 M NaCl) vorgelegt und 20 μl Amplifikat zupipettiert. Nach Anlegen eines Vakuums wurde gewartet, bis alle Flüssigkeit vollständig durchgesaugt war. Anschließend wurde mit 10 x SSC-Puffer nachgespült. Die Membran wurde, nachdem sie vollständig getrocknet war in einem UV-Crosslinker (UV-Stratalinker 2400, Stratagene, La Jolla, USA) bei 1200 Joule/cm$^2$ fixiert und mit destilliertem Wasser gewaschen und getrocknet.

Alle Hybridisierungen wurden bei 50°C in einem Hybridisierungsofen (Hybaid Mini Oven MkII, MWG-Biotech, Ebersberg, Deutschland) in Glasröhren durchgeführt. Die mit DNA/RNA-Amplifikat beschichtete Membran wurde in trockenem Zustand eingerollt und in eine Glasröhre gegeben. Anschließend wurde die Membran unter ständiger Rotation 5min mit vorgewärmten Hybridisierungspuffer inkubiert. Nach Zugabe von 2 pmol biotinilierter Sonde erfolgte für eine Stunde die Hybridisierungsreaktion. Nichtgebundene oder nur partial gebundene Sonde wurde durch 30min Inkubation mit Stringent-Puffer bei 50°C mit einmaligem Tausch des vorgewärmten Stringent-Puffers entfernt. Anschließend wurde Blocking-Reagens zugegeben und 15min bei 37°C weiterinkubiert. Die Hybride wurden durch ein Streptavidin-Alkalische Phosphatase-Konjugat durch Zugabe von NBT/BCIP kolorimetrisch oder durch Aufsprühen von Chemielumineszenzsubstrat (Lumi-Phos 530, Cellmark Diagnostics, Abindon, England) autoradiographisch detektiert. Dazu wurde Streptavidin-Alkalische Phosphatase-Konjugat zugegeben und 30min bei 37°C inkubiert. Anschließend wurde die Membran zweimal 15min mit Substratpuffer gewaschen. Die Membran wurde dann entnommen, Lumi-Phos-Reagens wurde aufgesprüht, gefolgt von 2h Exposition eines Röntgenfilms. Alternativ dazu wurde Substratpuffer mit NBT/BCIP zugegeben und die Farbentwicklung abgewartet.

Verwendete Lösungen:

10 x SSC-Lösung (Standard-Saline-Citrat):
1,5M NaCl, 0,15M Trinatriumcitrat;

Hybridisierungspuffer:
7% SDS (Na-dodecylsulfat), 0,25M Phosphatpuffer pH 7,5;

Stringentwaschlösung (Stringent-Puffer):
3M TMCL (Tetramethylammoniumchlorid), 50mM Tris/Cl, 2mM EDTA, 0,1% SDS;

Lösung zur Absättigung der Membranbindungsstellen:
5g/l Blockingreagenz (Roche) in Maleinsäurepuffer pH 7,5 (4,13g NaCl und 5,53g Maleinsäure in 500ml Wasser, pH mit 5M NaOH auf 7,5 eingestellt);

Substratpuffer:
274mM Tris/Cl pH 7,5, 68,6 mM Na$_3$Citrat, 200mM NaCl, 27,4 mM MgCl$_2$ * 6 H$_2$O;

BCIP:

50 mg/ml 5-bromo-4-chloro-3-indonylphosphat-toluidiniumsalz in 100% Dimethylformamid;

NBT:

75 mg/ml Nitroblautetrazoliumsalz in 70% Dimethylformamid;

Die Autoradiogramme wurden densitometrisch ausgewertet. Als 100%-Wert wurde der Amplifikatdot der Spezies, aus der die Sondensequenz abgeleitet wurde, zugrundegelegt. Als Kontrollen wurden immer eine Probe, der statt Nukleinsäurelösung Wasser zugegeben wurde und eine Probe mit 100ng isolierter humaner DNS als Dots auf der Membran mitgeführt.

Tabelle 2: Ergebnisse von Beispiel 1. Angegeben sind die %-Werte der densitometrischen Auswertung. Der Wert der für die Spezies homologen Sonde wurde 100% gesetzt. Die hier verwendeten Sonden können auch überlappende Spezifitäten haben.

| Sonde Taxon | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| M avi | 100.0 | 100.0 | 22.3 | 13.8 | 30.6 | 13.9 | 21.9 | 13.2 | 14.5 | 24.1 | 10.4 | 09.1 | 31.1 | 80.3 | 34.1 | 16.2 | 14.8 |
| M cel | 11.1 | 100.0 | 100.0 | 19.8 | 22.5 | 08.6 | 11.3 | 18.1 | 10.2 | 09.5 | 10.4 | 08.9 | 75.9 | 19.8 | 18.7 | 10.7 | 12.3 |
| M che | 14.0 | 75.4 | 21.1 | 100 0 | 11.2 | 14.8. | 10.4 | 20.7 | 12.9 | 08.4 | 07.3 | 12.3 | 17.6 | 19.3 | 82.0 | 23.9 | 10.2 |
| M fo1 | 19.1 | 92.9 | 11.1 | 17 8 | 100.0 | 22.6 | 40.7 | 09.5 | 15.0 | 08.9 | 09.4 | 11.3 | 19.2 | 11.9 | 19.6 | 10.8 | 08.4 |
| M.fo2 | 11.3 | 100.0 | 12.5 | 16 8 | 27.6 | 100.0 | 34.0 | 10.0 | 11.4 | 14.0 | 09.1 | 15.5 | 18.7 | 14.6 | 22.5 | 14.9 | 09.9 |
| M per | 17.3 | 100.0 | 24.6 | 19 4 | 14 2 | 11.4 | 100.0 | 22.6 | 14.6 | 24.6 | 21.4 | 19.8 | 19.2 | 10.2 | 21.6 | 28.0 | 14.6 |
| M gor | 08.6 | 100.0 | 17.4 | 21 4 | 02.3 | 10.9 | 14.6 | 100.0 | 04.8 | 14.6 | 17.5 | 14.1 | 20.1 | 01.6 | 88.2 | 09.9 | 10.4 |
| M mal | 08.4 | 94.2 | 12.6 | 19 7 | 09.6 | 11.4 | 14.2 | 17.5 | 100.0 | 10.2 | 10.8 | 18.4 | 12.6 | 17.6 | 74.3 | 09.7 | 12.4 |
| M phl | 14.6 | 100.0 | 09.5 | 04 6 | 06.4 | 05.5 | 08.4 | 08.1 | 03.5 | 100.0 | 01.9 | 09.6 | 11.8 | 09.8 | 04.1 | 05.3 | 02.4 |
| M tub | 17.8 | 88.6 | 11.5 | 18 1 | 20.1 | 14.2 | 12.6 | 18.4 | 14.6 | 14.9 | 100.0 | 16.2 | 21.4 | 14.6 | 82.4 | 24.5 | 10.0 |
| M xen | 04.6 | 100.0 | 02.4 | 03 7 | 02.6 | 01.8 | 05.4 | 01.2 | 01.6 | 01.6 | 01.7 | 100.0 | 01.4 | 02.0 | 02.1 | 01.4 | 01.8 |
| M.int | 19.5 | 100.0 | 21.4 | 19 4 | 26.3 | 25.4 | 19.2 | 29.1 | 24.6 | 20.0 | 34.7 | 18.9 | 100.0 | 100.0 | 41.4 | 15.0 | 19.7 |
| M scr | 20.1 | 97.3 | 19.4 | 24.6 | 27.4 | 20.3 | 20.6 | 20.1 | 22.9 | 17.5 | 32.4 | 19.2 | 100.0 | 29.1 | 78.9 | 15.5 | 17.8 |
| M itm | 21.5 | 100.0 | 17.1 | 22.1 | 28.4 | 25.4 | 20.1 | 20.9 | 29.3 | 19.4 | 38.4 | 24.6 | 100.0 | 34.0 | 100.0 | 11.8 | 19.8 |
| M kan | 09.1 | 79.3 | 08.5 | 09 4 | 08.1 | 09.9 | 10.2 | 08.6 | 07.4 | 14.1 | 07.4 | 04.6 | 18.4 | 09.8 | 92.9 | 100.0 | 07.1 |
| M.mar | 18.6 | 100.0 | 14.3 | 11.6 | 20.4 | 21.2 | 11.1 | 10.9 | 17.7 | 19.5 | 22.3 | 12.3 | 10.6 | 08.4 | 10.6 | 14.6 | 100.0 |
| E.col | 07.5 | 08.0 | 07.4 | 07.2 | 06.8 | 06.3 | 07.4 | 06.9 | 06.8 | 07.9 | 07.8 | 06.4 | 07.2 | 07.4 | 07.4 | 07.8 | 06.1 |
| hDNA | 0.1 | 0.2 | 0.02 | 0.04 | 0 06 | 0.1 | 0.06 | 0.1 | 0.06 | 0.01 | 0.2 | 0.5 | 0.04 | 0.1 | 0.06 | 0.02 | 0.08 |
| N-Kon | 0.04 | 0.02 | 0.01 | 0 01 | 0.02 | 0.02 | 0.1 | 0.1 | 0.06 | 0.04 | 0.09 | 0.2 | 0.06 | 0 05. | 0 01 | 0 01 | 0.04 |

Mit den hier beschriebenen Methoden können die entsprechenden Bakterien entweder aus Primärmaterial (z.B. Trachealsekrete, Wundabstriche, Blut u.ä.) oder aus bakteriellen Flüssig- oder Festmedien identifiziert und differenziert werden. Bei nicht primär sterilem Material muß zuvor die nicht säurefeste Begleitflora durch übliche

EP 1 390 541 B1

Dekontaminationsverfahren entfernt werden. Dies gilt sowohl für die Anlage der Kultur, als auch für die Vorbehandlung des Primärmaterials.

**Beispiel 2**

Mit den Methoden gemäß Beispiel 1 wurden weitere Proben mit Gram-positiven Bakterien mit hohem GC-Gehalt untersucht. Die untersuchten Bakterienspezies waren:

Tabelle 3:

| Bakterienspezies, die in Beispiel 2 untersucht wurden | | |
|---|---|---|
| Corynebacterium amycolatum | C.amy | ATCC Nr. |
| Corynebacterium callunae | C. cal | |
| Corynebacterium diphteriae gravis | C. dpg | |
| Corynebacterium glutamicum | C.glu | |
| CDC Coryneform Group G | C.grG | |
| Corynebacterium jeikeium | C.jei | |
| Corynebacterium matrucchotii | C.mat | |
| Corynebacterium minutissimun | C.min | |
| Corynebacterium pseudodiphteriticum | C.pdp | |
| Corynebacterium pseudotuberculosis | C.ptb | |
| Corynebacterium ulcerans | C.ulc | |
| Corynebacterium striatum | C.str | |
| Corynebacterium xerosis | C.xer | 373 |
| Nocardia asteroides | N.ast | |
| Rhodococcus equi | R.equ | |
| Tsukamurella paurometabolum | T.prm | |
| Escherichia coli | E.col | |
| Humane DNA | hDNA | |
| Negativkontrolle | N-Kon | |

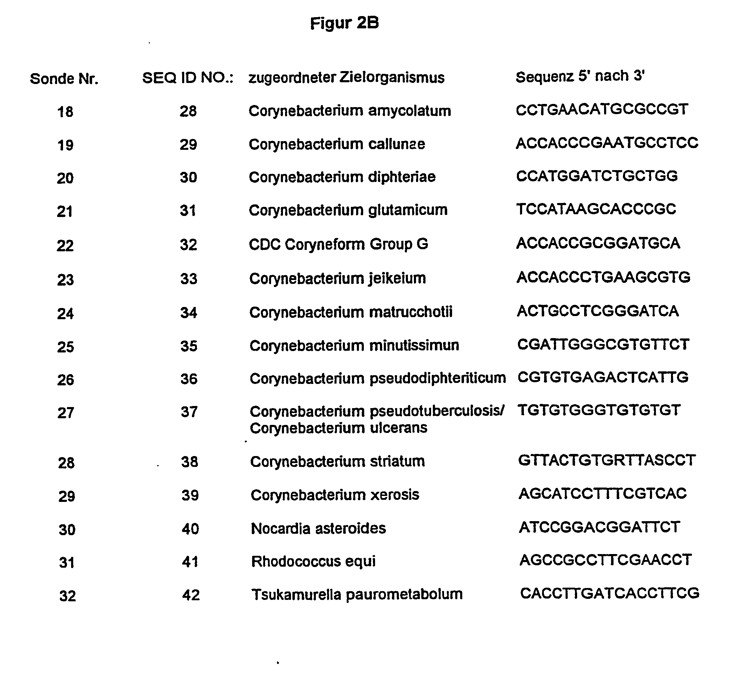

Als Sonden werden Oligonukleotide mit den Sequenzen SEQ ID NO. 28 bis 42 eingesetzt (siehe Figur 2B).
Die Auswertung erfolgte ebenfalls wie in Beispiel 1 beschrieben. Die Ergebnisse sind in Tabelle 4 zusammengefaßt.

Tabelle 4: Ergebnisse von Beispiel 2. Angegeben sind die %-Werte der densitometrischen Auswertung. Der Wert der für die Spezies homologen Sonde wurde 100% gesetzt. Die hier verwendeten Sonden können auch überlappende Spezifitäten haben.

| Sonde / Taxon | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|
| C.amy | 100,0 | 09,4 | 11,4 | 04,9 | 24,2 | 14,6 | 11,0 | 18,4 | 21,1 | 11,8 | 14,2 | 15,5 | 19,7 | 09,4 | 08,1 |
| C.cal | 19,5 | 100,0 | 11,7 | 18,8 | 20,6 | 20,0 | 11,8 | 14,6 | 14,5 | 16,4 | 19,2 | 12,1 | 11,5 | 16,6 | 18,4 |
| C.dpg | 14,4 | 16,5 | 100,0 | 12,7 | 17,6 | 13,7 | 14,4 | 19,4 | 20,0 | 26,1 | 09,7 | 11,1 | 17,6 | 13,2 | 17,1 |
| C.glu | 19,5 | 11,8 | 17,9 | 100,0 | 12,4 | 17,6 | 19,2 | 14,6 | 11,8 | 19,8 | 19,9 | 14,6 | 09,6 | 18,6 | 14,6 |
| C.grG | 21,9 | 12,8 | 18,2 | 18,3 | 100,0 | 15,8 | 20,3 | 11,6 | 14,4 | 15,9 | 22,1 | 13,6 | 17,6 | 11,2 | 16,9 |
| C.jei | 09,6 | 19,4 | 12,5 | 18,4 | 12,8 | 100,0 | 18,8 | 12,6 | 14,6 | 12,4 | 11,1 | 18,1 | 19,7 | 17,4 | 14,6 |
| C.mat | 15,4 | 13,6 | 13,8 | 12,7 | 17,6 | 16,5 | 100,0 | 10,9 | 11,7 | 11,2 | 13,5 | 15,6 | 17,2 | 12,5 | 13,7 |
| C.min | 16,5 | 14,6 | 13,8 | 17,6 | 12,6 | 12,1 | 20,5 | 100,0 | 15,4 | 16,4 | 17,6 | 17,4 | 12,5 | 04,6 | 22,9 |
| C.pdp | 15,2 | 16,8 | 17,1 | 11,2 | 20,1 | 08,6 | 15,4 | 14,9 | 100,0 | 08,9 | 16,4 | 18,4 | 13,8 | 14,6 | 20,1 |
| C.ptb | 14,6 | 14,6 | 18,6 | 17,6 | 14,2 | 14,3 | 16,5 | 14,8 | 16,7 | 100,0 | 23,4 | 11,3 | 10,9 | 17,3 | 26,1 |
| C.ulc | 16,4 | 16,9 | 21,4 | 19,6 | 15,4 | 13,5 | 17,2 | 17,5 | 17,4 | 100,0 | 24,9 | 12,3 | 10,0 | 19,1 | 27,4 |
| C.str | 05,6 | 19,4 | 14,6 | 12,4 | 18,4 | 16,9 | 18,3 | 12,1 | 14,1 | 16,2 | 100,0 | 10,2 | 10,9 | 19,5 | 20,4 |
| C.xer | 14,5 | 12,6 | 19,4 | 15,6 | 14,6 | 15,4 | 12,6 | 42,1 | 8 | 16,4 | 13,8 | 100,0 | 14,3 | 12,8 | 11,3 |
| N.ast | 07,4 | 06,1 | 09,2 | 10,3 | 04,2 | 03,6 | 08,8 | 04,6 | 09,5 | 10,8 | 02,6 | 11,5 | 100,0 | 06,5 | 05,2 |
| R.equ | 03,4 | 06,6 | 08,5 | 06,4 | 01,6 | 08,8 | 06,6 | 04,6 | 06,1 | 05,1 | 08,2 | 06,3 | 04,3 | 100,0 | 05,2 |
| T.prm | 04,3 | 02,6 | 08,5 | 02,9 | 09,1 | 06,6 | 08,3 | 08,2 | 08,6 | 01,6 | 05,4 | 06,4 | 04,2 | 03,6 | 100,0 |
| E.col | 06,5 | 05,3 | 07,1 | 06,5 | 04,2 | 04,2 | 09,1 | 07,2 | 01,4 | 05,4 | 04,1 | 02,2 | 06,5 | 05,1 | 04,2 |
| hDNA | 0,1 | 0,03 | 0,05 | 0,08 | 0,09 | 0,1 | 0,1 | 0,04 | 0,05 | 0,09 | 0,1 | 0,09 | 0,08 | 0,1 | 0,04 |
| N-Kon | 0,02 | 0,02 | 0,01 | 0,03 | 0,01 | 0,02 | 0,1 | 0,504 | 0,06 | 0,02 | 0,01 | 0,1 | 0,08 | 0,06 | 0,02 |

**Patentansprüche**

1. Verfahren zum Nachweis und/oder zur Identifizierung von Gram-positiven Bakterien mit hohem GC-Gehalt, **dadurch gekennzeichnet, daß** man

a) eine Probenzusammensetzung einer Nukleinsäureamplifikationsreaktion unterwirft, wobei ein erster Primer anwesend ist, der ausgewählt ist aus der Gruppe bestehend aus den Primem mit den Sequenzen SEQ ID NO. 1, SEQ ID NO. 3, SEQ ID NO. 5, SEQ ID NO. 7 und SEQ ID NO. 9, und ein zweiter Primer anwesend ist, der ausgewählt ist aus der Gruppe bestehend aus den Primem mit den Sequenzen SEQ ID NO. 2, SEQ ID NO. 4, SEQ ID NO. 6, SEQ ID NO. 8 und SEQ ID NO. 10;

b) eine Zusammensetzung, die das Amplifikationsprodukt aus Schritt a) oder einen Teil davon enthält, mit einer oder mehreren Sonden hybridisiert, die ausgewählt sind aus der Gruppe bestehend aus Oligonukleotiden mit den Sequenzen SEQ ID NO. 11 bis 42 und dazu komplementären Sequenzen; und

c) das Ausmaß der Hybridisierung bestimmt.

2. Verfahren nach Anspruch 1, **dadurch gekennzeichnet, daß** die Nukleinsäureamplifikationsreaktion eine Polymerasekettenreaktion ist.

3. Verfahren nach einem der vorhergehenden Ansprüche, **dadurch gekennzeichnet, daß** in der Nukleinsäureamplifikationsreaktion eines der Primerpaare mit den Sequenzen
SEQ ID NO. 1/SEQ ID NO. 2 (Primerpaar 1),
SEQ ID NO. 3/SEQ ID NO. 4 (Primerpaar 2),
SEQ ID NO. 5/SEQ ID NO. 6 (Primerpaar 3),
SEQ ID NO. 7/SEQ ID NO. 8 (Primerpaar 4) und
SEQ ID NO. 9/SEQ ID NO. 10 (Primerpaar 5)
anwesend ist.

4. Verfahren nach einem der vorhergehenden Ansprüche, **dadurch gekennzeichnet, daß** wenigstens eine Sonde oder ein Primer markiert ist.

**5.** Verfahren nach Anspruch 4, **dadurch gekennzeichnet, daß** die Markierung ausgewählt ist aus der Gruppe bestehend aus Fluoreszenzmarkierung, Biotin und Digoxigenin.

**6.** Verfahren nach Anspruch 4 oder 5, **dadurch gekennzeichnet, daß** wenigstens einer der Primer eine Markierung aufweist.

**7.** Verfahren nach Anspruch 6, **dadurch gekennzeichnet, daß** eine oder mehrere Sonden auf einer festen Phase immobilisiert werden und anschließend die feste Phase mit der Zusammensetzung, die das Amplifikationsprodukt aus Schritt a) oder einen Teil davon enthält, in Kontakt gebracht wird.

**8.** Verfahren nach Anspruch 7, **dadurch gekennzeichnet, daß** auf der festen Phase wenigstens zwei Sonden immobilisiert werden.

**9.** Verfahren nach Anspruch 7 oder 8, **dadurch gekennzeichnet, daß** auf der festen Phase die Sonden mit den Sequenzen SEQ ID NO. 11 bis 42 oder einer entsprechend komplementären Sequenz immobilisiert werden, wobei die einzelnen Sonden in räumlich voneinander getrennten Bereichen der festen Phase immobilisiert sind.

**10.** Verfahren nach Anspruch 4 oder 5, **dadurch gekennzeichnet, daß** wenigstens eine der Sonden eine Markierung aufweist.

**11.** Verfahren nach Anspruch 10, **dadurch gekennzeichnet, daß** das Amplifikationsprodukt oder ein Teil davon auf einer festen Phase immobilisiert wird und mit einer Zusammensetzung in Kontakt gebracht wird, die wenigstens eine Sonde enthält, die ausgewählt ist aus der Gruppe bestehend aus Sonden mit den Sequenzen SEQ ID NO. 11 bis 42 und dazu komplementären Sequenzen.

**12.** Kit zum Nachweis oder zur Identifizierung von Gram-positiven Bakterien mit hohem GC-Gehalt enthaltend

a) wenigstens ein Primerpaar, wobei der erste Primer ausgewählt ist aus der Gruppe bestehend aus den Primem mit den Sequenzen SEQ ID NO. 1, SEQ ID NO. 3, SEQ ID NO. 5, SEQ ID NO. 7 und SEQ ID NO. 9, und der zweite Primer ausgewählt ist aus der Gruppe bestehend aus den Primem mit den Sequenzen SEQ ID NO. 2, SEQ ID NO. 4, SEQ ID NO. 6, SEQ ID NO. 8 und SEQ ID NO. 10;

b) wenigstens ein Oligonukleotid mit einer Sequenz, die ausgewählt ist aus der Gruppe bestehend aus den Sequenzen SEQ ID NO. 11 bis 42 und dazu komplementären Sequenzen.

**13.** Kit nach Anspruch 13 enthaltend eines der Primerpaare
SEQ ID NO. 1/SEQ ID NO. 2 (Primerpaar 1),
SEQ ID NO. 3/SEQ ID NO. 4 (Primerpaar 2),
SEQ ID NO. 5/SEQ ID NO. 6 (Primerpaar 3),
SEQ ID NO. 7/SEQ ID NO. 8 (Primerpaar 4) und
SEQ 10 NO. 9/SEQ ID NO. 10 (Primerpaar 5).

**14.** Verwendung eines oder mehrerer der Oligonukleotide mit den Sequenzen SEQ ID NO. 1 bis 10 als Primer in einer Nukleinsäureamplifikationsreaktion, wobei die Nukleinsäureamplifikationsreaktion zum Nachweis und/oder zur Identifizierung von Gram-positiven Bakterien mit hohem GC-Gehalt durchgeführt wird.

**15.** Verwendung eines oder mehrerer der Oligonukleotide mit den Sequenzen SEQ ID NO. 11 bis 42 oder dazu komplementären Sequenzen als Sonde in einer Hybridisierungsreaktion, wobei die Hybridisierungsreaktion zum Nachweis und/oder zur Identifizierung von Gram-positiven Bakterien mit hohem GC-Gehalt durchgeführt wird.

**16.** DNA-Chip mit einer festen Phase, auf der die Oligonukleotide mit den Sequenzen SEQ ID NO. 11 bis 42 oder einer dazu komplementären Sequenz immobilisiert sind, wobei die verschiedenen Oligonukleotide räumlich voneinander getrennt sind.

**Claims**

**1.** A method for detecting and/or identifying gram-positive bacteria with high GC content, **characterized by**

a) subjecting a sample composition to a nucleic acid amplification reaction, with there being a first primer selected from the group consisting of the primers having the sequences SEQ ID NO. 1, SEQ ID NO. 3, SEQ ID NO. 5, SEQ ID NO. 7 and SEQ ID NO. 9 and there being a second primer selected from the group consisting of the primers having the sequences SEQ ID NO. 2, SEQ ID NO. 4, SEQ ID NO. 6, SEQ ID NO. 8 and SEQ ID NO. 10;

b) hybridizing a composition comprising the amplification product of step a) or a part thereof with one or more probes selected from the group consisting of oligonucleotides having the sequences SEQ ID NO. 11 to 42 and sequences complementary thereto; and

c) determining the degree of hybridization.

2. The method as claimed in claim 1, **characterized in that** the nucleic acid amplification reaction is a polymerase chain reaction.

3. The method as claimed in any of the preceding claims, **characterized in that** one of the primer pairs having the sequences
SEQ ID NO. 1/ SEQ ID NO. 2 (primer pair 1),
SEQ ID NO. 3/ SEQ ID NO. 4 (primer pair 2),
SEQ ID NO. 5/ SEQ ID NO. 6 (primer pair 3),
SEQ ID NO. 7/ SEQ ID NO. 8 (primer pair 4) and
SEQ ID NO. 9/ SEQ ID NO. 10 (primer pair 5
is present in the nucleic acid amplification reaction.

4. The method as claimed in any of the preceding claims, **characterized in that** at least one probe or one primer is labeled.

5. The method as claimed in claim 4, **characterized in that** the label is selected from the group consisting of fluorescent label, biotin and digoxigenin.

6. The method as claimed in claim 4 or 5, **characterized in that** at least one of the primers has a label.

7. The method as claimed in claim 6, **characterized in that** one or more probes are immobilized on a solid phase and said solid phase is then contacted with the composition comprising the amplification product of step a) or a part thereof.

8. The method as claimed in claim 7, **characterized in that** at least two probes are immobilized on the solid phase.

9. The method as claimed in claim 7 or 8, **characterized in that** the probes having the sequences SEQ ID NO. 11 to 42 or a correspondingly complementary sequence are immobilized on the solid phase, the individual probes being immobilized in spatially separated regions of said solid phase.

10. The method as claimed in claim 4 or 5, **characterized in that** at least one of the probes has a label.

11. The method as claimed in claim 10, **characterized in that** the amplification product or a part thereof is immobilized on a solid phase and contacted with a composition comprising at least one probe selected from the group consisting of probes having the sequences SEQ ID NO. 11 to 42 and sequences complementary thereto.

12. A kit for detecting or identifying gram-positive bacteria with high GC content, comprising

a) at least one primer pair, the first primer being selected from the group consisting of the primers having the sequences SEQ ID NO. 1, SEQ ID NO. 3, SEQ ID NO. 5, SEQ ID NO. 7 and SEQ ID NO. 9 and the second primer being selected from the group consisting of the primers having the sequences SEQ ID NO. 2, SEQ ID NO. 4, SEQ ID NO. 6, SEQ ID NO. 8 and SEQ ID NO. 10;

b) at least one oligonucleotide having a sequence selected from the group consisting of the sequences SEQ ID NO. 11 to 42 and sequences complementary thereto.

**13.** The kit as claimed in claim 12, comprising any of the primer pairs
SEQ ID NO. 1/ SEQ ID NO. 2 (primer pair 1),
SEQ ID NO. 3/ SEQ ID NO. 4 (primer pair 2),
SEQ ID NO. 5/ SEQ ID NO. 6 (primer pair 3),
SEQ ID NO. 7/ SEQ ID NO. 8 (primer pair 4) and
SEQ ID NO. 9/ SEQ ID NO. 10 (primer pair 5).

**14.** The use of one or more of the oligonucleotides having the sequences SEQ ID NO. 1 to 10 as primers in a nucleic acid amplification reaction, said nucleic acid amplification reaction being carried out to detect and/or identify gram-positive bacteria having a high GC content.

**15.** The use of one or more of the oligonucleotides having the sequences SEQ ID NO. 11 to 42 or sequences complementary thereto as probe in a hybridization reaction, said hybridization reaction being carried out to detect and/ or identify gram-positive bacteria having a high GC content.

**16.** DNA chip having a solid phase, on which the oligonucleotides having the sequences SEQ ID NO. 11 to 42 or a sequence complementary thereto are immobilized, the various oligonucleotides being spatially separated from one another.

**Revendications**

**1.** Procédé de détection et/ou d'identification de bactéries Gram positif avec teneur élevée en GC, **caractérisé en ce que**

a) on soumet une composition échantillon à une réaction d'amplification de l'acide nucléique, où une première amorce est présente, qui est choisie parmi le groupe constitué des amorces avec les séquences SEQ ID N°1, SEQ ID N°3, SEQ ID N°5, SEQ ID N°7 et SEQ ID N°9, et une deuxième amorce est présente, qui est choisie parmi le groupe constitué des amorces avec les séquences SEQ ID N°2, SEQ ID N°4, SEQ ID N°6, SEQ ID N°8 et SEQ ID N°10 ;
b) on hybride une composition, qui contient le produit d'amplification de l'étape a) ou une partie de celui-ci, avec une ou plusieurs sondes, qui sont choisies parmi le groupe consistant en les oligonucléotides ayant les séquences SEQ ID N°11 à 42 et leurs séquences complémentaires, et
c) on détermine la mesure de l'hybridation.

**2.** Procédé selon la revendication 1, **caractérisé en ce que** la réaction d'amplification de l'acide nucléique est une réaction en chaîne par polymérase.

**3.** Procédé selon l'une des revendications précédentes, **caractérisé en ce que**, dans la réaction d'amplification de l'acide nucléique, est présent une des paires d'amorces ayant les séquences
SEQ ID N°1/SEQ ID N°2 (paire d'amorces 1),
SEQ ID N°3/SEQ ID N°4 (paire d'amorces 2),
SEQ ID N°5/SEQ ID N°6 (paire d'amorces 3),
SEQ ID N°7/SEQ ID N°8 (paire d'amorces 4), et
SEQ ID N°9/SEQ ID N°10 (paire d'amorces 5).

**4.** Procédé selon l'une des revendications précédentes, **caractérisé en ce qu'**au moins une sonde ou une amorce est marquée.

**5.** Procédé selon la revendication 4, **caractérisé en ce que** le marquage est choisi parmi le groupe constitué du marquage par fluorescence, par la biotine et par la digoxigénine.

**6.** Procédé selon la revendication 4 ou 5, **caractérisé en ce qu'**au moins une des amorces présente un marquage.

**7.** Procédé selon la revendication 6, **caractérisé en ce qu'**une ou plusieurs sondes sont immobilisées sur une phase solide et ensuite, la phase solide est mise en contact avec la composition, qui contient le produit d'amplification de l'étape a) ou une partie de celui-ci.

**8.** Procédé selon la revendication 7, **caractérisé en ce que** l'on immobilise au moins deux sondes sur la phase solide.

**9.** Procédé selon la revendication 7 ou 8, **caractérisé en ce que** l'on immobilise sur la phase solide, les sondes ayant les séquences SEQ ID N°11 à 42 ou une séquence complémentaire correspondante, chaque sonde étant immobilisée dans des zones spatialement séparées les unes des autres, de la phase solide.

**10.** Procédé selon la revendication 4 ou 5, **caractérisé en ce qu'**au moins une des sondes présente un marquage.

**11.** Procédé selon la revendication 10, **caractérisé en ce que** le produit d'amplification ou une partie de celui-ci est immobilisé sur une phase solide, et est mis en contact avec une composition qui contient au moins une sonde, qui est choisie parmi le groupe constitué des sondes ayant les séquences SEQ ID N°11 à 42 et leurs séquences complémentaires.

**12.** Nécessaire de détection ou d'identification de bactéries Gram positif avec teneur élevée en GC, contenant :

a) au moins une paire d'amorces, où la première amorce est choisie parmi le groupe constitué des amorces avec les séquences SEQ ID N°1, SEQ ID N°3, SEQ ID N°5, SEQ ID N°7 et SEQ ID N°9, et la deuxième amorce est choisie parmi le groupe constitué des amorces avec les séquences SEQ ID N°2, SEQ ID N°4, SEQ ID N°6, SEQ ID N°8 et SEQ ID N°10 ;
b) au moins un oligonucléotide avec une séquence qui est choisie parmi le groupe constitué des séquences SEQ ID N°11 à 42 et leurs séquences complémentaires.

**13.** Nécessaire selon la revendication 12, contenant une des paires d'amorces
SEQ ID N°1/SEQ ID N°2 (paire d'amorces 1),
SEQ ID N°3/SEQ ID N°4 (paire d'amorces 2),
SEQ ID N°5/SEQ ID N°6 (paire d'amorces 3),
SEQ ID N°7/SEQ ID N°8 (paire d'amorces 4),
SEQ ID N°9/SEQ ID N°10 (paire d'amorces 5).

**14.** Utilisation d'un ou de plusieurs des oligonucléotides ayant les séquences SEQ ID N°1 à 10, comme amorce dans une réaction d'amplification de l'acide nucléique, où la réaction d'amplification de l'acide nucléique est réalisée pour la détection et/ou pour l'identification de bactéries Gram positif avec teneur élevée en GC.

**15.** Utilisation d'un ou de plusieurs des oligonucléotides ayant les séquences SEQ ID N°11 à 42 ou leurs séquences complémentaires, comme sonde dans une réaction d'hybridation, la réaction d'hybridation étant réalisée pour la détection et/ou pour l'identification de bactéries Gram positif avec teneur élevée en GC.

**16.** Puce à ADN avec une phase solide, sur laquelle les oligonucléotides ayant les séquences SEQ ID N°11 à 42 ou leurs séquences complémentaires sont immobilisées, les différents oligonucléotides étant spatialement séparés les uns des autres.

**Figur 1**

| | | SEQ ID NO.: |
|---|---|---|
| Vorwärtsprimer Nr. 1: | GTC GAT GGA CAA CGG GTT GAT (1854-F) | 1 |
| Vorwärtsprimer Nr. 2: | GCG GTA CTA ACC ACC CAA AA (1915-F) | 3 |
| Vorwärtsprimer Nr. 3: | GGACCTAAGGCGAGGCCG (54F) | 5 |
| Vorwärtsprimer Nr. 4: | AGTGAGAATGCAGGCATGAG (1726) | 7 |
| Vorwärtsprimer Nr. 5: | GCGTAATAGCTCACT (1023) | 9 |
| | | |
| Rückwärtsprimer Nr. 1: | GGTACGGCTACCTTCCTGCGTCA (2052) | 2 |
| Rückwärtsprimer Nr. 2: | TTACCACTGACTGGTACGGCTA (2049) | 4 |
| Rückwärtsprimer Nr. 3: | GCCTTCCTGCGTCACCCC (2025) | 6 |
| Rückwärtsprimer Nr. 4: | CTTAGGATGGTTATAGTTACCA ( HCG II ) | 8 |
| Rückwärtsprimer Nr. 5: | CGGAACTTACCCGA | 10 |

## Figur 2A

| Sonde Nr. | SEQ ID NO.: | zugeordneter Zielorganismus | Sequenz 5' nach 3' |
|---|---|---|---|
| 1 | 11 | Mycobacterium avium | GCRTGGCGATTCGGG |
| 2 | 12 | Gram + mit hohem GC-Gehalt | CGACTACGCCTGTCGG |
| 3 | 13 | Mycobacterium celatum | GCACGGTCAGCGAGG |
| 4 | 14 | Mycobacterium chelonae | AGCGGGTTCACGTCG |
| 5 | 15 | Mycobacterium fortuitum 1 | GGATCAGTCACGACG |
| 6 | 16 | Mycobacterium fortuitum 2 | TGGTCACGGTGGTTT |
| 7 | 17 | Mycobacterium peregrinum | GGATCGGTCACGACG |
| 8 | 18 | Mycobacterium gordonae | TAGCACAATTGATTC |
| 9 | 19 | Mycobacterium malmoense | GTGGAGGTTCGGGGC |
| 10 | 20 | Mycobacterium phlei | ATCGGCCAGGTTTTG |
| 11 | 21 | Mycobacterium tuberculosis-Komplex | GGAGTTCTGGGGCTG |
| 12 | 22 | Mycobacterium xenopi | TTAGCAGATCCGATT |
| 13 | 23 | Mycobacterium intracellulare/ Mycobacterium scrophulaceum Mycobacterium interjectum | CCCCGAAACTCCAYGC |
| 14 | 24 | Mycobacterium intracellulare | ACGCSCATACACGGG |
| 15 | 25 | Mycobacterium scrophulaceum Mycobacterium interjectum | ACGCSCATATACGGG |
| 16 | 26 | Mycobacterium kansasii | GGGGCGTGGAGGTCT |
| 17 | 27 | Mycobacterium marinum | CAACGAACGTTCCAC |

## Figur 2B

| Sonde Nr. | SEQ ID NO.: | zugeordneter Zielorganismus | Sequenz 5' nach 3' |
|---|---|---|---|
| 18 | 28 | Corynebacterium amycolatum | CCTGAACATGCGCCGT |
| 19 | 29 | Corynebacterium callunae | ACCACCCGAATGCCTCC |
| 20 | 30 | Corynebacterium diphteriae | CCATGGATCTGCTGG |
| 21 | 31 | Corynebacterium glutamicum | TCCATAAGCACCCGC |
| 22 | 32 | CDC Coryneform Group G | ACCACCGCGGATGCA |
| 23 | 33 | Corynebacterium jeikeium | ACCACCCTGAAGCGTG |
| 24 | 34 | Corynebacterium matrucchotii | ACTGCCTCGGGATCA |
| 25 | 35 | Corynebacterium minutissimun | CGATTGGGCGTGTTCT |
| 26 | 36 | Corynebacterium pseudodiphteriticum | CGTGTGAGACTCATTG |
| 27 | 37 | Corynebacterium pseudotuberculosis/ Corynebacterium ulcerans | TGTGTGGGTGTGTGT |
| 28 | 38 | Corynebacterium striatum | GTTACTGTGRTTASCCT |
| 29 | 39 | Corynebacterium xerosis | AGCATCCTTTCGTCAC |
| 30 | 40 | Nocardia asteroides | ATCCGGACGGATTCT |
| 31 | 41 | Rhodococcus equi | AGCCGCCTTCGAACCT |
| 32 | 42 | Tsukamurella paurometabolum | CACCTTGATCACCTTCG |

SEQUENZPROTOKOLL

<110> Hain Diagnostica GmbH

<120> Verfahren zum Nachweis von gram-positiven Bakterien

<130> Hain

<140>
<141>

<160> 42

<170> PatentIn Ver. 2.1

<210> 1
<211> 21
<212> DNA
<213> Künstliche Sequenz

<220>
<223> Beschreibung der künstlichen Sequenz: Primer

<400> 1

```
gtcgatggac aacgggttga t                                             21
```

<210> 2
<211> 23
<212> DNA
<213> Künstliche Sequenz

<220>
<223> Beschreibung der künstlichen Sequenz: Primer

<400> 2

```
ggtacggcta ccttcctgcg tca                                           23
```

<210> 3
<211> 20
<212> DNA
<213> Künstliche Sequenz

<220>
<223> Beschreibung der künstlichen Sequenz: Primer

<400> 3

```
gcggtactaa ccacccaaaa                                               20
```

<210> 4
<211> 22
<212> DNA
<213> Künstliche Sequenz

<220>
<223> Beschreibung der künstlichen Sequenz: Primer

<400> 4

```
ttaccactga ctggtacggc ta                                            22
```

<210> 5
<211> 18
<212> DNA
<213> Künstliche Sequenz

<220>
<223> Beschreibung der künstlichen Sequenz: Primer

<400> 5

```
ggacctaagg cgaggccg                                                 18
```

<210> 6
<211> 18
<212> DNA
<213> Künstliche Sequenz

<220>
<223> Beschreibung der künstlichen Sequenz: Primer

<400> 6

```
gccttcctgc gtcacccc                                                 18
```

<210> 7
<211> 20
<212> DNA
<213> Künstliche Sequenz

<220>
<223> Beschreibung der künstlichen Sequenz: Primer

<400> 7

```
agtgagaatg caggcatgag                                               20
```

<210> 8

<211> 22
<212> DNA
<213> Künstliche Sequenz

<220>
<223> Beschreibung der künstlichen Sequenz: Primer

<400> 8

`cttaggatgg ttatagttac ca`                                                                 `22`

<210> 9
<211> 15
<212> DNA
<213> Künstliche Sequenz

<220>
<223> Beschreibung der künstlichen Sequenz: Primer

<400> 9

`gcgtaatagc tcact`                                                                         `15`

<210> 10
<211> 14
<212> DNA
<213> Künstliche Sequenz

<220>
<223> Beschreibung der künstlichen Sequenz: Primer

<400> 10

`cggaacttac ccga`                                                                          `14`

<210> 11
<211> 15
<212> DNA
<213> Künstliche Sequenz

<220>
<223> Beschreibung der künstlichen Sequenz: Sonde

<400> 11

`gcrtggcgat tcggg`                                                                         `15`

<210> 12
<211> 16
<212> DNA

<213> Künstliche Sequenz

<220>
<223> Beschreibung der künstlichen Sequenz: Sonde

<400> 12

```
cgactacgcc tgtcgg                                                    16
```

<210> 13
<211> 15
<212> DNA
<213> Künstliche Sequenz

<220>
<223> Beschreibung der künstlichen Sequenz: Sonde

<400> 13

```
gcacggtcag cgagg                                                     15
```

<210> 14
<211> 15
<212> DNA
<213> Künstliche Sequenz

<220>
<223> Beschreibung der künstlichen Sequenz: Sonde

<400> 14

```
agcgggttca cgtcg                                                     15
```

<210> 15
<211> 15
<212> DNA
<213> Künstliche Sequenz

<220>
<223> Beschreibung der künstlichen Sequenz: Sonde

<400> 15

```
ggatcagtca cgacg                                                     15
```

<210> 16
<211> 15
<212> DNA
<213> Künstliche Sequenz

<220>
<223> Beschreibung der künstlichen Sequenz: Sonde

<400> 16

   tggtcacggt ggttt                                 **15**

<210> 17
<211> 15
<212> DNA
<213> Künstliche Sequenz

<220>
<223> Beschreibung der künstlichen Sequenz: Sonde

<400> 17

   ggatcggtca cgacg                                 **15**

<210> 18
<211> 15
<212> DNA
<213> Künstliche Sequenz

<220>
<223> Beschreibung der künstlichen Sequenz: Sonde

<400> 18

   tagcacaatt gattc                                 **15**

<210> 19
<211> 15
<212> DNA
<213> Künstliche Sequenz

<220>
<223> Beschreibung der künstlichen Sequenz: Sonde

<400> 19

   gtggaggttc ggggc                                 **15**

<210> 20
<211> 15
<212> DNA
<213> Künstliche Sequenz

<220>
<223> Beschreibung der künstlichen Sequenz: Sonde

<400> 20

```
atcggccagg ttttg                        .                                    15
```

.

<210> 21
<211> 15
<212> DNA
<213> Künstliche Sequenz

<220>
<223> Beschreibung der künstlichen Sequenz: Sonde

<400> 21

```
ggagttctgg ggctg                                 .                           15
```

<210> 22
<211> 15
<212> DNA
<213> Künstliche Sequenz

<220>
<223> Beschreibung der künstlichen Sequenz: Sonde

<400> 22

```
ttagcagatc cgatt                                                             15
```

<210> 23
<211> 16
<212> DNA
<213> Künstliche Sequenz

<220>
<223> Beschreibung der künstlichen Sequenz: Sonde

<400> 23

```
ccccgaaact ccaygc                                                            16
```

<210> 24
<211> 15
<212> DNA
<213> Künstliche Sequenz

<220>
<223> Beschreibung der künstlichen Sequenz: Sonde

<400> 24

```
acgcscatac acggg                                    15
```
.

<210> 25
<211> 15
<212> DNA
<213> Künstliche Sequenz

<220>
<223> Beschreibung der künstlichen Sequenz: Sonde

<400> 25

```
acgcscatat acggg                                    15
```

<210> 26
<211> 15
<212> DNA
<213> Künstliche Sequenz

<220>
<223> Beschreibung der künstlichen Sequenz: Sonde

<400> 26

```
ggggcgtgga ggtct                                    15
```

<210> 27
<211> 15
<212> DNA
<213> Künstliche Sequenz

<220>
<223> Beschreibung der künstlichen Sequenz: Sonde

<400> 27

```
caacgaacgt tccac                                    15
```

<210> 28
<211> 16
<212> DNA
<213> Künstliche Sequenz

<220>
<223> Beschreibung der künstlichen Sequenz: Sonde

<400> 28

```
cctgaacatg cgccgt                                   16
```
.

<210> 29
<211> 17
<212> DNA
<213> Künstliche Sequenz

<220>
<223> Beschreibung der künstlichen Sequenz: Sonde

<400> 29

```
accacccgaa tgcctcc                                                    17
```

<210> 30
<211> 15
<212> DNA
<213> Künstliche Sequenz

<220>
<223> Beschreibung der künstlichen Sequenz: Sonde

<400> 30

```
ccatggatct gctgg                                                      15
```

<210> 31
<211> 15
<212> DNA
<213> Künstliche Sequenz

<220>
<223> Beschreibung der künstlichen Sequenz: Sonde

<400> 31

```
tccataagca cccgc                                                      15
```

<210> 32
<211> 15
<212> DNA
<213> Künstliche Sequenz

<220>
<223> Beschreibung der künstlichen Sequenz: Sonde

<400> 32

```
accaccgcgg atgca                                                      15
```

<210> 33

<211> 16
<212> DNA
<213> Künstliche Sequenz

<220>
<223> Beschreibung der künstlichen Sequenz: Sonde

<400> 33

accaccctga agcgtg                                                        16

<210> 34
<211> 15
<212> DNA
<213> Künstliche Sequenz

<220>
<223> Beschreibung der künstlichen Sequenz: Sonde

<400> 34

actgcctcgg gatca                                                         15

<210> 35
<211> 16
<212> DNA
<213> Künstliche Sequenz

<220>
<223> Beschreibung der künstlichen Sequenz: Sonde

<400> 35

cgattgggcg tgttct                                                        16

<210> 36
<211> 16
<212> DNA
<213> Künstliche Sequenz

<220>
<223> Beschreibung der künstlichen Sequenz: Sonde

<400> 36

cgtgtgagac tcattg                                                        16

<210> 37
<211> 15

<212> DNA
<213> Künstliche Sequenz

<220>
<223> Beschreibung der künstlichen Sequenz: Sonde

<400> 37

`tgtgtgggtg tgtgt` 15

<210> 38
<211> 17
<212> DNA
<213> Künstliche Sequenz

<220>
<223> Beschreibung der künstlichen Sequenz: Sonde

<400> 38

`gttactgtgr ttascct` 17

<210> 39
<211> 16
<212> DNA
<213> Künstliche Sequenz

<220>
<223> Beschreibung der künstlichen Sequenz: Sonde

<400> 39

`agcatccttt cgtcac` 16

<210> 40
<211> 15
<212> DNA
<213> Künstliche Sequenz

<220>
<223> Beschreibung der künstlichen Sequenz: Sonde

<400> 40

`atccggacgg attct` 15

<210> 41
<211> 16
<212> DNA
<213> Künstliche Sequenz

<220>
<223> Beschreibung der künstlichen Sequenz: Sonde

<400> 41

**agccgccttc gaacct** . **16**

<210> 42
<211> 17
<212> DNA
<213> Künstliche Sequenz

<220>
<223> Beschreibung der künstlichen Sequenz: Sonde

<400> 42

**caccttgatc accttcg** **17**